# EUROPEAN PATENT APPLICATION

(11) **EP 4 759 234 A1**
(43) Date of publication of application: **17.06.2026**
(21) Application number: 25305317.7
(22) Date of filing: 10.03.2025
(51) Int. Cl.: A61B 5/384, A61B 5/00

(54) **DEVICE FOR GENERATING A PRETRAINED FOUNDATION MODEL, DEVICE FOR OBTAINING A REPRESENTATION OF AN INPUT EEG SEGMENT AND RELATED METHODS**

(71) Applicant: Bioserenity Medical Devices Group, 75013 Paris (FR)
(72) Inventor: RAHMOUNI, Mohamed, 75013 Paris (FR); BETTINARDI, Ruggero, 75013 Paris (FR); GIMENEZ, Ulysse, 75013 Paris (FR)
(74) Representative: Icosa

(57) **Abstract**

The present invention relates to a device (1) for generating a pretrained foundation model (31) for obtaining a representation (33) of an input EEG segment (32) previously acquired, said device comprising:
- at least one input,
- _at least one processor configured to generate said pretrained foundation model (31) via a training process based on said training dataset and comprising a first phase and a second phase,
- at least one output configured to provide as output said pretrained foundation model (31).

## Description

### FIELD OF INVENTION

The present invention relates to the field of electroencephalographic signals processing.

More specifically, the present invention relates to a device for generating a pretrained foundation model for obtaining a representation of an input EEG segment, a device for obtaining a representation of an input EEG segment, and associated methods.

### BACKGROUND OF INVENTION

Electroencephalography (EEG) is a non-invasive technique that measures and records the brain's electrical activity through electrodes placed on the scalp. This technology captures the electrical signals generated by neurons communicating via synaptic excitations, providing real-time insights into brain function. This has made EEG a valuable source of information enabling applications in multiple domains. In the medical field, EEG has been shown to be able to provide detailed and relevant information in several health conditions, such as epilepsy and seizure disorders, sleep disorders, neurodegenerative diseases as Alzheimer, Parkinson and other forms of dementia, traumatic brain injuries and psychiatric conditions. Impressive advances have also been made using EEG to develop brain-computer interfaces (BCI), devices that leverage the brain's electrical potentials recorded at the level of the scalp to guide and accelerate rehabilitation for patients affected by stroke or spinal cord injuries, wheelchair control for paralyzed individuals, to build communication systems for locked-in patients, as well as a plethora of non-medical applications ranging from emotion recognition and cognitive state monitoring to entertainment and gaming interfaces.

Early attempts at automatizing the interpretation of the EEG signal relied on manually defining signal features later used to perform analyses and classification of the EEG. However, the identification and definition of these features is a non-trivial task even for experienced professionals. The EEG is in fact a very complex type of signal emerging by the simultaneous activity and interplay of tens of thousands of neurons at different spatial and temporal scales, characterized by periodic and aperiodic components, large inter-subject and intra-subject variability and multiple sources of artifacts and interference, leading to poor signal-to-noise ratio.

Modern analysis approaches employ various deep learning architectures, as they enable end-to-end processing of EEG signals, eliminating the need for manual feature extraction. This provide several, ground-breaking advantages over other approaches: direct learning from raw EEG data, improving model accuracy and efficiency, automatic detection of hidden, meaningful temporal and spatial patterns in noisy signals that might be missed by other methods.

The amount of data used to pre-train the referenced models vary from less than 2000 to more than 40,000 hours of EEG signal, coming from diverse sources: clinical EEG, signals recorded from brain-computer interface experiments (BCI) or research experiments (emotion recognition, speech processing, ...). The EEG systems used, and EEG channels also varied widely across the referenced models, as the downstream tasks used to evaluate the pretrained models' ability to generalize to previously unseen data and contexts.

There is a need for improving the performances of deep learning models currently used for EEG signals analysis and EEG signals-based prediction tasks. The invention aims at improving the situation.

### SUMMARY

The present invention relates to a device for generating a pretrained foundation model for obtaining a representation of an input EEG segment previously acquired, said device comprising:
- at least one input configured to receive:
   - a training dataset comprising a plurality of EEG segments previously acquired, each EEG segment comprising the EEG signals from multiple channels obtained by a subject from plurality of subjects,
   - an untrained encoder, being associated to a codebook, configured to receive as input a set of input patches associated to an input EEG segment and to provide as output a set of embedding vectors each representative of one input patch in a latent representation space (i.e., representation of the at least one input EEG segment), said set of input patches being extracted from said input EEG segment,
   - a untrained decoder configured to receive as input a set of codebook vectors assigned to said set of embedding vectors associated to said input EEG segment and to output a set of power spectra corresponding to said set of input patches,
- at least one processor configured to generate said pretrained foundation model via a training process based on said training dataset,
- at least one output configured to provide as output said pretrained foundation model, wherein said training process comprises:
   - obtaining a pretrained encoder and an updated codebook by training the untrained encoder and untrained decoder and updating the codebook using said training dataset,
   - obtaining said pretrained foundation model by performing self-supervised learning using said training dataset, said untrained encoder, said pretrained encoder, said updated codebook and a predefined loss function; wherein the pretrained foundation model comprises the untrained encoder with updated weights obtained at the end of the self-supervised learning.

According to one embodiment, obtaining a pretrained encoder and an updated codebook by training the untrained encoder and untrained decoder and updating the codebook using said training dataset, comprises performing iteratively until convergence, for each EEG segment of said plurality of EEG segments:
- obtaining a set of patches, each patch being extracted from the EEG segment;
- providing said set of patches as input to said untrained encoder and obtaining as output a set of embedding vectors;
- mapping said set of embedding vectors to the codebook so to obtain the associated set of codebook vectors using quantization;
- decoding with the untrained decoder said set of codebook vectors, and
- updating the codebook and updating weights of the untrained encoder and the untrained decoder.

According to one embodiment, obtaining said pretrained foundation model by performing self-supervised learning comprises performing iteratively until convergence, for each EEG segment of said plurality of EEG segments:
- obtaining an unmasked set of patches, each patch of the unmasked set of patches being extracted from the EEG segment;
- providing said unmasked set of patches as input to said pretrained encoder and obtaining as output a set of target encoded patches and mapping them to the updated codebook obtaining a set of target indices associated to a set of target codebook vectors associated to the EEG segment;
- obtaining a masked set of patches by masking at least one patch of said unmasked set of patches;
- providing said masked set of patches as input to said untrained encoder and obtaining as output a set of predicted encoded patches and mapping them to the updated codebook obtaining a set of predicted indices associated to a set of predicted codebook vectors associated to the EEG segment;
- updating weights of the untrained encoder;

wherein said predefined loss function is configured to compare the set of target indices with the set of predicted indices,
wherein the pretrained foundation model comprises the untrained encoder with said updated weights obtained at the end of the self-supervised learning.

According to one embodiment, the device comprises:
- at least one input configured to receive:
- a training dataset comprising a plurality of EEG segments previously acquired, each EEG segment comprising the EEG signals from multiple channels obtained by a subject from plurality of subjects,
- an untrained encoder, being associated to a codebook, configured to receive as input a set of input patches associated to an input EEG segment and to provide as output a set of embedding vectors each representative of one input patch in a latent representation space, said set of input patches being extracted from said input EEG segment,
- a untrained decoder configured to receive as input a set of codebook vectors assigned to said set of embedding vectors associated to said input EEG segment and to output a set of power spectra corresponding to said set of input patches,
- at least one processor configured to generate said pretrained foundation model via a training process based on said training dataset,
- at least one output configured to provide as output said pretrained foundation model, wherein said training process comprises:
- obtaining a pretrained encoder and an updated codebook by training the untrained encoder and untrained decoder and updating the codebook using said training dataset, by performing iteratively until convergence, for each EEG segment of said plurality of EEG segments:
   - obtaining a set of patches, each patch being extracted from the EEG segment;
   - providing said set of patches as input to said untrained encoder and obtaining as output a set of embedding vectors;
   - mapping said set of embedding vectors to the codebook so to obtain the associated set of codebook vectors using quantization;
      - decoding with the untrained decoder said set of codebook vectors, and
   - updating the codebook and updating weights of the untrained encoder and the untrained decoder;
   - obtaining said pretrained foundation model by performing self-supervised learning using said training dataset, said untrained encoder, said pretrained encoder, said updated codebook and a predefined loss function; wherein said self-supervised learning comprises performing iteratively until convergence, for each EEG segment of said plurality of EEG segments:
      - obtaining an unmasked set of patches, each patch of the unmasked set of patches being extracted from the EEG segment;
      - providing said unmasked set of patches as input to said pretrained encoder and obtaining as output a set of target encoded patches and mapping them to the updated codebook obtaining a set of target indices associated to a set of target codebook vectors associated to the EEG segment;
      - obtaining a masked set of patches by masking at least one patch of said unmasked set of patches;
      - providing said masked set of patches as input to said untrained encoder and obtaining as output a set of predicted encoded patches and mapping them to the updated codebook obtaining a set of predicted indices associated to a set of predicted codebook vectors associated to the EEG segment;
      - updating weights of the untrained encoder;

   wherein said predefined loss function is configured to compare the set of target indices with the set of predicted indices;
   wherein the pretrained foundation model comprises the untrained encoder with the updated weights obtained at the end of the self-supervised learning;
- at least one output configured to provide as output said pretrained foundation model

Advantageously, by leveraging a first architecture during training that is based on an encoder and a decoder configured to output power spectra of patches of EEG segments, an effective suppression of the influence of non-stationarities and artifacts commonly encountered in electrophysiological data is achieved. Using the reconstruction of power spectra instead of raw EEG signals as a proxy task for learning latent representations takes advantage of the fact that the spectral representation of an EEG signal tends to be more stable over time than the raw EEG signal itself, which is inherently characterized by a low signal-to-noise ratio. This makes power spectra more reliable targets for self-supervised learning.

Besides, the training process based on a first phase (i.e., obtaining a pretrained encoder and an updated codebook) and on a second phase (i.e., obtaining the pretrained foundation model by performing self-supervised learning) effectively pushes the pretrained foundation model to learn generalizable features of the input space of EEG segments, relying on both local and global relationships between different channels and temporal segments.

In addition, the pretrained foundation model obtained from the device according to the present invention demonstrates superior performance in reconstructing codebook vectors representing an input EEG segment, which significantly enhances downstream tasks.

In some embodiments, the representation of said input EEG segment is configured to be used as input of at least one fine-tuned machine learning model related to a predefined task, said at least one predefined task being one among:
- a discrimination of said input EEG segment between a normal EEG segment and an abnormal EEG segment,
- an anesthesia depth determination,
- a pathology classification task,
- a sleep staging task,
- seizure detection task,
- EEG biomarker identification.

Therefore, the pretrained foundation model obtained with the device according to the present invention can serve a diverse number of applications. In addition, the use of the representation obtained from the pretrained foundation model advantageously allows reducing the volumetry of EEG data used to train a machine learning model related to a specific downstream task, as compared to classic monobloc models currently used for EEG signals analysis.

In some embodiments:
- the at least one input is further configured to receive a specific training dataset comprising labelled specific training EEG segments being specific to said downstream task,
- the training process further comprises a fine-tuning phase based on said specific training dataset.

In some embodiments:
- the untrained encoder comprises an architecture comprising convolutional layers, time and channel encoding and several transformer blocks, each transformer block comprising attention heads and,
- the fine-tuning phase comprises training a said at least one machine learning model upended to said pretrained foundation model, which is used as base model.

In some embodiments, the untrained encoder and the untrained decoder are comprised in a vector-quantization variational autoencoder architecture.

Another aspect of the invention relates to a device for obtaining a representation of an input EEG segment, by using a pretrained foundation model obtained with a device as previously described and comprising:
- at least one input configured to receive:
- at least one EEG segment;
- at least one processor configured to:
- extract a set of input patches from said at least one EEG segment;
- provide, as input to said pretrained foundation model, said set of input patches,
- obtain, as output, a set of indices associated to a set of codebook vectors corresponding to said set of input patches,
- extract a set of embedding vectors using said set of indices and an updated codebook of the pretrained foundation model,
- at least one output configured to provide said representation of said input EEG segment, wherein said representation of the at least one input EEG segment is defined as said set of embedding vectors.

Therefore, as previously mentioned, the device according to the present invention allows obtaining a faithful and robust representation of an input EEG segment.

Another aspect of the invention relates to a computer-implemented method for generating a pretrained foundation model for obtaining a representation of an input EEG segment previously acquired, said method comprising:
- receiving:
   - a training dataset comprising a plurality of EEG segments previously acquired, each EEG segment comprising the EEG signals from multiple channels obtained by a subject from plurality of subjects,
   - an untrained encoder, being associated to a codebook, configured to receive as input a set of input patches associated to an input EEG segment and to provide as output a set of embedding vectors each representative of one input patch in a latent representation space, said set of input patches being extracted from said input EEG segment,
   - a untrained decoder configured to receive as input a set of codebook vectors assigned to said set of embedding vectors associated to said input EEG segment and to output a set of power spectra corresponding to said set of input patches,
- at least one processor configured to generate said pretrained foundation model via a training process based on said training dataset,
- providing as output said pretrained foundation model,
   wherein said training process comprises:
   - obtaining a pretrained encoder and an updated codebook by training the untrained encoder and untrained decoder and updating the codebook using said training dataset, by performing iteratively until convergence, for each EEG segment of said plurality of EEG segments:
      - obtaining a set of patches, each patch being extracted from the EEG segment;
      - providing said set of patches as input to said untrained encoder and obtaining as output a set of embedding vectors;
      - mapping said set of embedding vectors to the codebook so to obtain the associated set of codebook vectors using quantization;
      - decoding with the untrained decoder said set of codebook vectors, and
      - updating the codebook and updating weights of the untrained encoder and the untrained decoder;
   - obtaining said pretrained foundation model by performing self-supervised learning using said training dataset, said untrained encoder, said pretrained encoder, said updated codebook and a predefined loss function; wherein said self-supervised learning comprises performing iteratively until convergence, for each EEG segment of said plurality of EEG segments:
      - obtaining an unmasked set of patches, each patch of the unmasked set of patches being extracted from the EEG segment;
      - providing said unmasked set of patches as input to said pretrained encoder and obtaining as output a set of target encoded patches and mapping them to the updated codebook obtaining a set of target indices associated to a set of target codebook vectors associated to the EEG segment;
      - obtaining a masked set of patches by masking at least one patch of said unmasked set of patches;
      - providing said masked set of patches as input to said untrained encoder and obtaining as output a set of predicted encoded patches and mapping them to the updated codebook obtaining a set of predicted indices associated to a set of predicted codebook vectors associated to the EEG segment;
   - updating weights of the untrained encoder;

wherein said predefined loss function is configured to compare the set of target indices with the set of predicted indices;
wherein the pretrained foundation model comprises the untrained encoder with the updated weights obtained at the end of the self-supervised learning.

As previously mentioned, the use of the representation obtained from the pretrained foundation model according to the present invention, compact while faithful, improves the performances of a fine-tuned machine learning model related to a specific task.

In some embodiments, the at least one predefined task is one among:
- a discrimination of said input EEG segment between a normal EEG segment and an abnormal EEG segment,
- an anesthesia depth determination,
- a pathology classification task,
- a sleep staging task,
- seizure detection task,
- EEG biomarker identification.

Another aspect of the present invention relates to a use of a representation of an input EEG segment obtained with the method as previously described, as input of a downstream task being a discrimination of the input EEG segment between a normal segment and an abnormal segment.

Another aspect of the present invention relates to a use of a representation of an input EEG segment obtained with the method as previously described, as input of a downstream task being a discrimination of said input EEG segment between a pathology classification task.

In addition, the disclosure relates to a computer program product comprising instructions which, when the program is executed by a computer, cause the computer to carry out the methods compliant with any of the above execution modes.

The present disclosure further pertains to a computer-readable storage medium comprising instructions which, when executed by a computer, cause the computer to carry out the methods compliant with any of the above execution modes.

The present disclosure further relates to a non-transitory program storage device (i.e. computer-readable storage medium), readable by a computer, tangibly embodying a program of instructions executable by the computer to perform the computer-implemented methods, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:

The terms **"adapted"** and **"configured"** are used in the present disclosure as broadly encompassing initial configuration, later adaptation or complementation of the present device, or any combination thereof alike, whether effected through material or software means (including firmware).

The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

**"Machine learning (ML)"** designates in a traditional way computer algorithms improving automatically through experience, on the ground of training data enabling to adjust parameters of computer models through gap reductions between expected outputs extracted from the training data and evaluated outputs computed by the computer models.

### BRIEF DESCRIPTION OF THE DRAWINGS

**Figure 1** is a block diagram representing schematically a particular mode of a device 1 for generating a pretrained foundation model 31 for obtaining a representation of an input EEG segment.
**Figure 2** is a schematic representation of a first architecture 311 to be trained during a first phase 14 of a training process according to the present disclosure
**Figure 3** is an example of an architecture of an untrained encoder 21 of the first architecture 311 of Figure 2.
**Figure 4** is an example of an architecture of an untrained decoder 22 of the first architecture 311 of Figure 2
**Figure 5** is a schematic representation of a second architecture 312 to be trained during a second phase 15 of a training process according to the present disclosure
**Figure 6** is an example of steps to be implemented in a first phase 14 of a training process according to the present disclosure.
**Figure 7** is an example of steps to be implemented in a second phase 15 of a training process according to the present disclosure.
**Figure 8** is a flow chart showing successive steps executed with the device 1 for generating a pretrained foundation model 31 for obtaining a representation of an input EEG segment illustrated on Figure 1.
**Figure 9** is a block diagram representing schematically a particular mode of a device 2 for obtaining a representation of an input EEG segment.
**Figure 10** is a flow chart showing successive steps for predicting according to a predefined task using a pretrained foundation model 31 obtained from a method illustrated on Figure 8.
**Figure 11** is a flow chart showing successive steps executed with the device 2 for obtaining a representation of an input EEG segment, illustrated on Figure 9
**Figure 12** shows performance metrics of a first phase 14 of a training process according to the present disclosure.
**Figure 13** shows a comparison of original patches spectra with patches spectra reconstructed with a first architecture 311 trained after a first phase 14 of a training process according to the present disclosure.
**Figure 14** shows performance metrics of a second phase 15 of a training process according to the present disclosure.
**Figure 15** shows a comparison of performances obtained with a pretrained foundation model 31 according to the present disclosure and state of the art models across a downstream specific task consisting of seizure detection.
**Figure 16** shows a comparison of performances obtained with a pretrained foundation model 31 according to the present disclosure and state of the art models across a downstream specific task consisting of normal vs. abnormal EEG classification using a first specific training dataset.
**Figure 17** shows a comparison of performances obtained with a pretrained foundation model 31 according to the present disclosure and state of the art models across a downstream specific task consisting of normal vs. abnormal EEG classification using a second specific training dataset.
**Figure 18** shows a comparison of performances obtained with a pretrained foundation model 31 according to the present disclosure and state of the art models across a downstream specific task consisting of multi-class EEG classification.

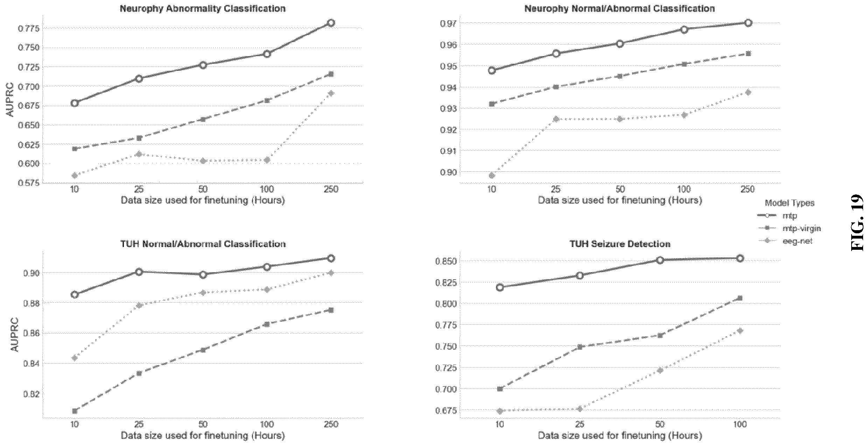

**Figure 19** shows the performances of a pretrained foundation model 31 according to the present disclosure in low-data regimes.

### DETAILED DESCRIPTION

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

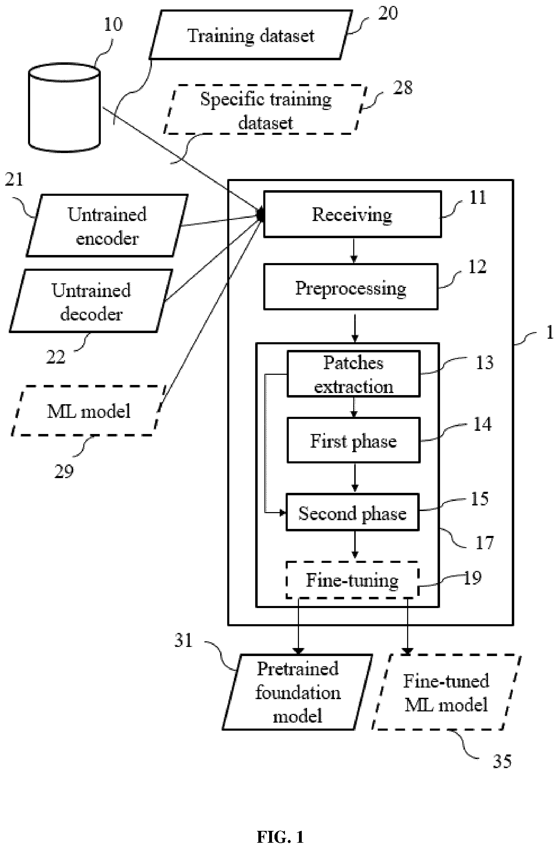

The present disclosure will be described in reference to a particular functional embodiment of a device 1 for generating a pretrained foundation model 31 for obtaining a representation of an input EEG segment previously acquired, as illustrated on Figure 1.

The device 1 is adapted to generate a pretrained foundation model 31 for obtaining a representation of an input EEG segment 32 previously acquired. Said pretrained foundation model 31 is configured to receive, as an input, a set of input patches 24, obtained from the input EEG segment 32, and generate as output a representation 33 of the input EEG segment 32 (e.g., a set of embedding vectors 34).

Notably, the device 1 is configured to train, using a training dataset 20, a first architecture 311 and a second architecture 312, during respectively a first phase 14 and a second phase 15. The first architecture 311 comprises an untrained encoder 21 associated to a codebook 23, and an untrained decoder 22, while the second architecture 312 comprised an identical untrained encoder 21, to obtain the pretrained foundation model 31. In other words, the pretrained foundation model 31 corresponds to the second architecture 312 trained after the second phase 15. The term "untrained" referred to a machine learning model, such as an untrained encoder or untrained decoder, refers to a model that has been initialized but has not yet undergone any training process. This means that its parameters (e.g., weights and biases in a model) are set to their initial values, which may have been randomly assigned or predefined based on a specific initialization strategy (e.g., Xavier or He initialization).

As will be seen further, advantageously the pretrained foundation model 31 allows obtaining a representation of an input EEG segment 32 that can improve the performance of a machine learning model 29 configured to perform a predetermined downstream task.

In some embodiments, the representation 33 of the input EEG segment 32 is configured to be used as input of at least one machine learning model 29 fine-tuned, into a fine-tuned machine learning model 35, on a predefined task. The at least one predefined task may be chosen among:
- a discrimination of said input EEG segment between a normal EEG segment and an abnormal EEG segment,
- an anesthesia depth determination,
- a pathology classification task,
- a sleep staging task,
- seizure detection task,
- EEG biomarker identification.

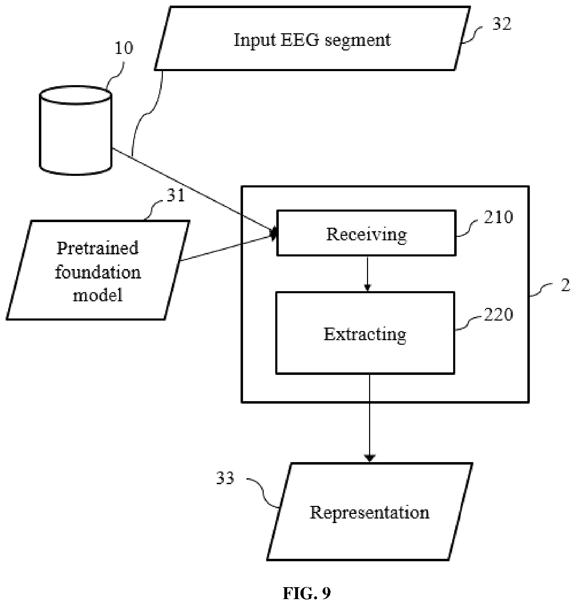

The device 1 for generating a pretrained foundation model 31 (i.e. trained so as to set the pretrained foundation model parameters) may be associated with a device 2, represented on Figure 9. The device 2 is a device for obtaining a representation of an input EEG segment 32 previously acquired, from the input EEG segment 3, by using the pretrained foundation model 31 obtained from the device 1, which will be subsequently described.

Though the presently described devices 1 and 2 are versatile and provided with several functions that can be carried out alternatively or in any cumulative way, other implementations within the scope of the present disclosure include devices having only parts of the present functionalities.

Each of the devices 1 and 2 is advantageously an apparatus, or a physical part of an apparatus, designed, configured and/or adapted for performing the mentioned functions and produce the mentioned effects or results. In alternative implementations, any of the device 1 and the device 2 is embodied as a set of apparatus or physical parts of apparatus, whether grouped in a same machine or in different, possibly remote, machines. The device 1 and/or the device 2 may e.g. have functions distributed over a cloud infrastructure and be available to users as a cloud-based service, or have remote functions accessible through an API.

The device 1 for generating and the device 2 for obtaining a representation 33 of an input EEG segment 32 previously acquired may be integrated in a same apparatus or set of apparatus, and intended to same users. In other implementations, the structure of the device 2 may be completely independent of the structure of the device 1, and may be provided for other users. For example, the device 2 may comprise the pretrained foundation model 31 available to operators for obtaining a representation of an input EEG segment previously acquired, wholly set from previous training effected upstream by other players with the device 1.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1 or of the device 2.

### Description of the inputs of device 1

The device 1 comprises a module 11 for receiving at least the training dataset 20, the untrained encoder 21 and the untrained decoder 22, stored in one or more local or remote database(s). The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

In the present disclosure, the training dataset 20 comprises a plurality of EEG segments previously acquired. Each EEG segment 32 comprising the EEG signals from multiple channels obtained by a subject from a plurality of subjects. Each EEG segment comprises at least two EEG signals, each corresponding to one distinct channel of an electroencephalographic (EEG) device. As the EEG segments are previously acquired the implementation of the methods and devices of the present invention do not implies an practice on a subject body.

For instance, the multiple channels can be a subset of channels from a 10-20 montage system. For instance, those multiple channels comprise the following channels: FP1, FP2, F7, F3, F4, F8, T7, C3, C4, T8, P7, P3, P4, P8, O1, and O2.

In some embodiments, the multiple channels comprise at least 16 channels with a minimal duration of 16 seconds.

In some embodiments, the minimum sampling frequency of the EEG signals is 128 Hz.

### Preprocessing and input patches extraction from an EEG segment of the training dataset 20

In some embodiments, the device 1 further comprises a module 12 configured for preprocessing data from the training dataset 20. One of the goals of data preprocessing is to ensure signal quality and standardization across EEG recordings.

In some embodiments, an initial frequency filtering is performed on the at least one input EEG segment. For instance, the initial frequency filtering involves involved a high-pass filter at 0.5 Hz and a low-pass filter at 45 Hz.

In some embodiments, each EEG segment of the plurality of EEG segments is further downsampled to 128 Hz to reduce computational load while preserving relevant neurophysiological information.

In some embodiments, to ensure consistency across all recordings while maximizing the number of records available for pre-training, only a predetermined of channels common to all records were retained. It is recalled that each EEG segment comprises EEG signals from multiple channels obtained from a subject among a plurality of subjects and is of shape (T_seg * fs, nbChannels), with T_seg the length of the segment (for instance in seconds), fs the sampling frequency, and nbChannels the number of channels. An example of shape is (2048, 16). For instance, 16 channels systematically arranged in the following order to preserve spatial information are retained: FP1, FP2, F7, F3, F4, F8, T7, C3, C4, T8, P7, P3, P4, P8, O1, and O2.

In some embodiments, the average signal is then removed from each channel to reduce the effects of noise sources common to all channels and standardize reference across channels.

In some embodiments, the EEG of each record is then divided into non-overlapping windows of 16 seconds so to obtain one or more EEG segments. For instance, each segment is composed of 16 channels by 16 seconds = 256 patches (one patch being defined as the EEG signal of one channel during one second, i.e. a vector of 128-time samples).

In some embodiments, multiple windows can then be combined into larger data "shards", used to efficiently distribute the workload, for instance on multiple processing units in a distributed data parallel (DDP) framework for use during a training process that will be described below.

### Training process

The device 1 further comprises a module 17 configured to train the first architecture 311 and the second architecture 312 by a training process which uses the training dataset 20 received by the module 11.

In some embodiments, the training process comprises a first phase 14 and a second phase 15 succeeding the first phase 14 that will be described below.

The present training process may notably use a "patch-based learning" or "patch-wise training". As illustrated in Figure 1, module 17 may comprise a sub-module 13 configured to obtain a set of patches, each patch being extracted from the EEG segment. The set of patches may comprise at least two patches or at least three patches and may be obtained by further segmenting the EEG segment 32 into sub-segments (e.g. using a time window of length inferior to the EEG segments length) and by associating one patch to one channel of the multiple channels comprised in the EEG segment. In one example, if an EEG segment consists of 16 channels and spans 16 seconds, module 12 is configured to apply a 1-second time window to extract sub-segments resulting in 256 input patches. Each input patch may have a shape dependent from the number of channels, the length of the time windows used for segmentation and sampling frequency. An example of input patch shape is (16, 16, 128). In other words, one input patch is defined as the EEG signal of one channel during one second, i.e. a vector of 128-time samples.

### Description of the architecture of the pretrained foundation model 31

Module 17 may be further configured to comprise a sub-module 14 configured to perform the first phase of training and a sub-module 15 configured to perform the second phase, which involve, as previously mentioned, each, a different architecture, referred to as first architecture and second architecture.

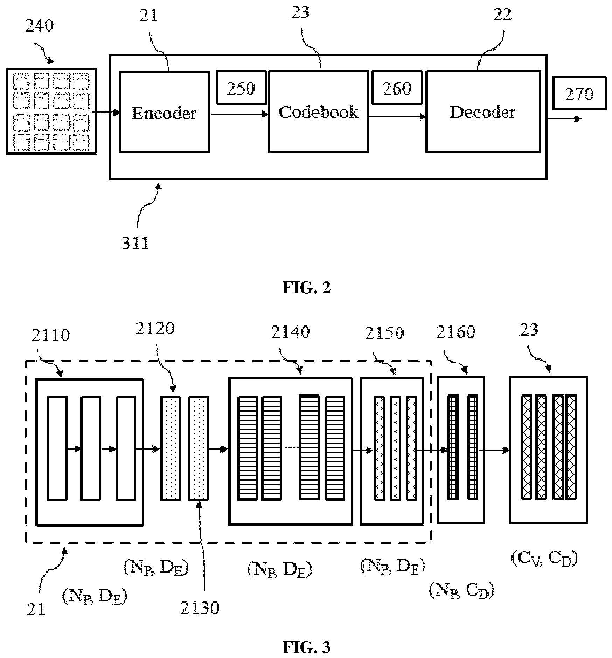

More in detail, in some embodiments, the first architecture 311, illustrated on Figure 2, comprises the untrained encoder 21, which associated to a codebook 23, and the untrained decoder 22. The untrained encoder 21 is configured to receive as input a set of patches 240 associated to an EEG segment 32 of the plurality of EEG segments previously acquired, and to provide as output a set of embedding vectors 250 each representative of one input patch in a latent representation space. The set of patches 240 are extracted from the EEG segment by sub-module 13 as described above.

In one example, module 17 may be configured so that the set of input patches 240 is provided as a sequence of Np patches, where Np is defined as follows. Each EEG segment of the plurality of EEG segments, is of the shape (Tw*fs, Nc), with Tw a time window, fs the sampling frequency of the signal, and Nc the number of channels. The sequence of Np patches is a reshaping of each time window and comprises Np equal to Nc*Tw patches.

The untrained decoder 22 is configured to receive as input a set of codebook vectors 260 assigned to the set of embedding vectors 250 associated to the EEG segment and to output a set of power spectra 270 corresponding to the set of input patches 240 (e.g., the number of power spectra in the set is equal to the number of patched in the set of input patches 240).

In some embodiments, the untrained encoder 21 comprises a set of convolutional layers 2110 for performing temporal encoding, channel encoding layers 2120, time encoding layers 2130 and a set of transformer blocks 2140. Each transformer block comprises attention heads. Figure 3 illustrates an untrained encoder architecture according to those embodiments.

In the example of Figure 3, the temporal encoding consists of consecutive 1-D CNNs with group normalization and GELU activation functions, to extract temporal features of structure (nbChannels, T_seg, embed_dim), with nbChannels the number of channels, T_seg is the length of the time window used to obtain the patches, embed_dim, the dimensions of each output of the temporal encoding. For instance, nbChannels is equal to 16, T_seg is equal to 16, and embed_dim is equal to 64.

In the example of Figure 3, the positional encoding (i.e. the channel encoding layers 2120, configured to encode positional and channel information) comprises embedding layers of structure (nbChannels * T_seg, embed_dim), and implementing sinusoidal position encoding. With the previous example values of nbChannels, T_seg and embed_dim, the embedding layers may have a structure (256, 64).

In one example, the set of transformer blocks 2140 comprise a sequence of 12 transformer blocks. Each of the transformer blocks comprises 8 attention heads. The attention heads further enrich the patch encoding by using a self-attention mechanism. The set of transformer blocks 2140 will produce a set of embedding vectors 2150. Each patch will be summarized by an embedding vector of D_{E} numbers. In an example, each input patch (originally representing 1 second of an EEG signal corresponding to 1 channel) will be summarized by 64 numbers. The shape of the set of embedding vectors is then (256, 64).

In some embodiments, drop out and normalization are applied to the set of embedding vectors, providing an output of dimensions (N_{P}, D_{E}). In other words, the set of embedding vectors produces a whole encoded sequence 2150 of dimensions (N_{P}, D_{E}).

For example, the first architecture, comprising the untrained encoder 21, the untrained decoder 22, may be comprised in a vector-quantization variational autoencoder architecture comprising a VQ-VAE codebook.

In this example, the association between the untrained encoder 21 and the codebook 23 takes the form of a mapping of each 64-dimensional encoded vector to the nearest latent embedding vector in the VQ-VAE codebook by using a quantization process. For instance, cosine similarity can be used for the mapping.

For instance, as illustrated on Figure 3, the quantization process comprises mapping each encoded patch into vectors of C_{D} elements through two densely connected layers 2160, then mapped to the nearest latent embedding vector in the codebook 23 based on cosine similarity. This produces a quantized sequence with shape (N_{P}, C_{D}).

The codebook 23 is then a matrix of C_{V} embedding vectors (codebook size) each defined by C_{D} elements (embedding vector dimension). For instance, C_{D} is equal to 8192 and C_{D} is equal to 64. In other terms, the number C_{D} corresponds to the number of discrete latent representations of different power spectrum distributions the encoder 21 is aimed to be able to recognize, and the number C_{V} is the number of abstract dimensions on which each latent vector is to be encoded. The quantization process provides with the set of codebook vectors 260.

In some embodiments, the untrained decoder 22 is configured to reconstruct, based on the set of codebook vectors 260, the Power Spectral Density (PSD) of the EEG signal within each patch.

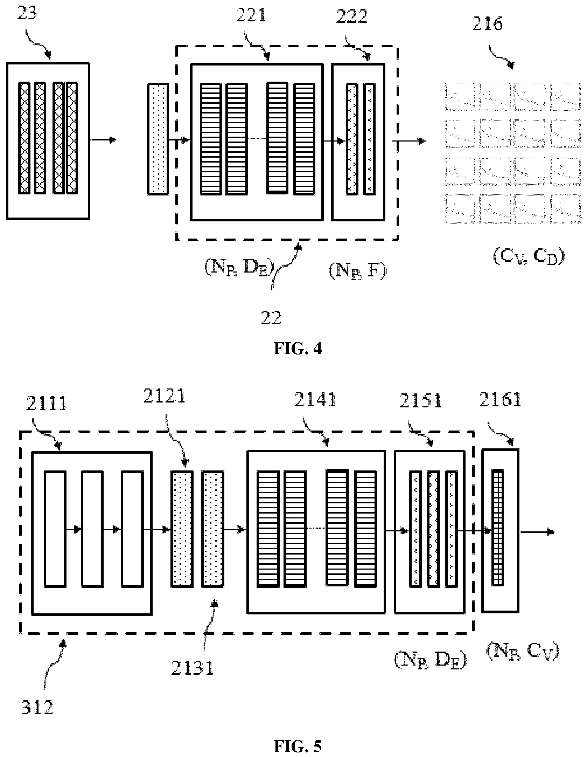

For instance, the untrained decoder 22 can comprise a mirror architecture of the untrained encoder 21. For instance, as illustrated on Figure 4, the untrained decoder 22 can comprise a sequence of transformer blocks 221, each comprising attention heads as the encoder transformer blocks 2140. The untrained decoder 22 may have a reduced depth as compared to the untrained encoder 21. In addition, the untrained decoder 22 may comprise, after the sequence of transformer blocks 221, a prediction head 222 configured to project the output of the transformer layers so as to reconstruct the power spectra of all Np input patches. The power spectra are of the shape (Np, F) with F the number of spectral frequencies.

As an example, the untrained decoder 22 can comprise 3 layers versus 12 in the untrained encoder 21).

In some embodiments, the second architecture 312 is identical to the architecture of the untrained encoder 21 of the first architecture 311.

In those embodiments, as illustrated on Figure 5, the second architecture 312 comprises a set of convolutional layers 2111 for performing temporal encoding, channel encoding layers 2121, time encoding layers 2131 and a set of transformer blocks 2141. Each transformer block comprises attention heads.

Module 17 is therefore configured to implement the first phase 14 and the second phase 15 using the above-described architectures. Sub-module 14 is configured to train the untrained encoder 21 and untrained decoder 22 and updating the codebook (i.e., first architecture 311) using said training dataset 20.

More in detail, sub-module 14 may be to train the first architecture 311 based on the training dataset 20 by optimizing a predefined reconstruction loss function until convergence by implementing a backpropagation gradient algorithm. At the end of the first phase 14, an updated codebook UC is obtained. One goal of the first phase 14 is to learn a way to compress the input EEG segment into a fixed number of meaningful features able to "summarize" its most relevant aspects in a way that they can generalize to unseen EEG data (i.e., EEG segments). As such, the outcome of the first phase 14 can be seen as a way to project the continuous EEG input into a common lower-dimensional discrete subspace (the "codebook") that reduces the dimensionality of the input while preserving its more relevant inner relationships.

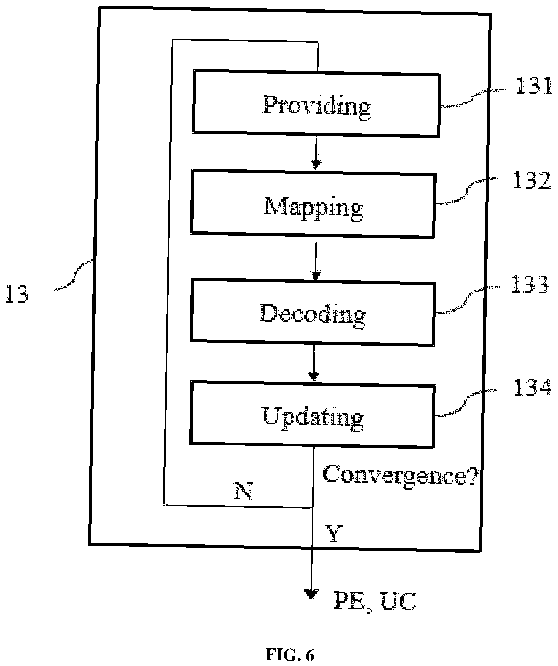

The first phase of training may comprise the iteration of the following steps until convergence, as illustrated on Figure 6:
- providing a set of patches 24 as input to the untrained encoder 21 and obtaining as output a set of embedding vectors 250 (step 131);
- mapping said set of embedding vectors 250 to the codebook 23 so to obtain the associated set of codebook vectors 260 using quantization (step 132);
- decoding with the untrained decoder 22 the set of selected codebook vectors 260 (step 133), and
- updating the codebook 23 and updating weights of the untrained encoder 21 and the untrained decoder 23 (step 134).

At step 131, a set of patches 124 is provided as input to the untrained encoder 21. The set of patches 124 can be supplied as a sequence of Np patches, where Np is defined as follows. Each EEG segment of the plurality of EEG segments of the training dataset 21, is of the shape (Tw*fs, Nc), with Tw a time window, fs the sampling frequency of the signal, and Nc the number of channels. The sequence of Np patches is a reshaping of each time window and comprises Np equal to Nc*Tw patches.

In some embodiments, the logarithm of the estimated power spectrum distribution is used to minimize the reconstruction loss function, as it rescales the power distribution in a way that enhances differences across frequencies in a range meaningful for EEG analysis in clinical contexts, for instance between 1 and 45 Hz.

In some embodiments, the output of the decoder 22 is used to calculate the predefined reconstruction loss function against the power spectrum of each patch using Discrete Prolate Spheroidal Sequences (DPSS) multitaper windowing.

Throughout the iterations, the codebook 23 is updated alongside the first architecture weights through back-propagation (i.e., the weight of the encoder 21 and decoder 22 are updated).

When the first architecture 311 comprises a vector-quantization variational autoencoder, the latter is trained to learn to encode each EEG segment into a discrete codebook, i.e. a latent subspace of arbitrary shape that summarizes the most relevant information needed to reconstruct the power spectrum of the encoded EEG signal via a decoder block.

At the end of the first phase 14, a pretrained encoder PE and an updated codebook UC are obtained. More specifically, the updated codebook UC will then be used in the second phase 15 as ground-truth to train the second architecture 312.

In some embodiments, sub-module 15 is configured to implement the second phase 15 so as to train the second architecture 312 in the following manner, by performing self-supervised learning using the training dataset 20, the second architecture 312 (i.e., untrained encoder), the pretrained encoder PE, the updated codebook UC and a second predefined loss function. The second phase 15 is based on the use of masked patches and aims at training the second architecture 312 to identify codebook indices corresponding to codebook vectors assigned to those masked patches.

In some embodiments, the second phase 15 aims at providing the pretrained foundation model 31. The pretrained foundation model 31 comprises the second architecture 312 trained after the second phase 15. In other words, the pretrained foundation model 31 comprises the untrained encoder with the updated weights obtained at the end of the self-supervised learning.

According to one embodiment, the sub-module 13 is further configured to obtain said unmasked set of patches, wherein each patch of the unmasked set of patches being extracted from the EEG segment. The patches may be extracted as described above, and the set of masked patches is obtained by selecting a subset of patches and masking them. The proportion of patches (e.g., 30%-50%) may be randomly selected for masking. The selected patches may be removed or replaced (e.g., with a learned mask token, noise, or zeros). In one example, the at least one input EEG segment, being of the shape (Tw*fs, Nc), with Tw a time window, fs, the sampling frequency of the signal, and Nc the number of channels, is segmented so as to extract a sequence of Np patches, where Np equal to Nc*Tw patches.

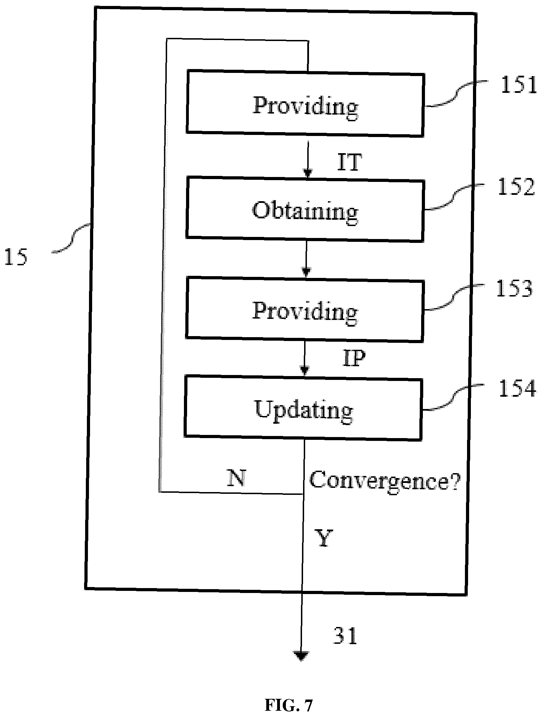

More specifically, sub-module 15 is configured to perform iteratively the following steps iteratively until convergence, for each EEG segment of the plurality of EEG segments of the training dataset 20, as illustrated on Figure 7:
- providing the unmasked set of patches as input to the pretrained encoder PE and obtaining as output a set of target encoded patches and mapping them to the updated codebook UC, obtaining a set of target indices IT associated to the a set of target codebook vectors associated to the EEG segment (step 151);
- obtaining a masked set of patches by masking at least one patch of the at least two patches of said unmasked set of patches (step 152);

- providing the masked set of patches as input to the second architecture 312 and obtaining as output a set of predicted encoded patches and mapping them to the updated codebook UC obtaining a set of predicted indices IP associated to a set of predicted codebook vectors associated to the EEG segment (step 153);
- updating weights of the second architecture 312 (step 154).

More in details, the step 152 of providing the unmasked set of patches as input to the pretrained encoder PE amounts to passing this unmasked set of patches to a portion of the first architecture up to the set of transformer blocks 2140 of the pretrained encoder PE, so as to obtain a set of embedding vectors that will serve as the ground truth.

At step 152, the unmasked set of patches is passed through the temporal encoding layers 2111 of the second architecture 312 producing a set of embeddings. Then, a given portion of those embeddings (in other words, a subset of those embeddings) are randomly substituted via a learnable mask token initialized with small random values drawn from a normal distribution, resulting in a tensor storing both masked and non-masked patch embeddings. The tensor thus contains partially masked embeddings. The tensor is then passed through the rest of the second architecture 312, starting from the positional encoding layers 2121 and up to the transformer layers coupled 2141 to GELU activations functions.

In some embodiments, downstream from the second architecture 312, a multi-layer perceptron head 2161 is used and configured to project the output of the set of transformer blocks 2141 into logits of dimensions (Np, C_{V}). Then, the logits are converted into the embedding vectors prediction space by extracting a codebook index associated to the largest logit for each patch. Those extracted codebook indices are referred to as predicted indices IP.

Advantageously, hyperparameters of the ensemble formed by the second architecture 312 and the multi-layer perceptron head 2161 can be tuned so as to tailor the final size of the pretrained foundation model 31. For instance, such parameters as embed_dim, size of the outputs of the set of convolutional layers 2111, encoder depth, multi-layer perceptron head size "MLP size" can be chosen so as to obtain a given size for the pretrained foundation model 31. In a first example, the hyperparameters embed_dim, encoder depth, MLP size are respectively equal to 192, 6 and 768. In a second example, the hyperparameters embed_dim, encoder depth, MLP size are respectively equal to 256, 12 and 1024. In a third example, the hyperparameters embed_dim, encoder depth, MLP size are respectively equal to 384, 18 and 1536.

The second predefined loss function is configured to compare the set of target indices IT with the set of predicted indices IP.

In some embodiments, the second predefined loss function is a cross-entropy loss function between the target indices IT associated to the embedding vectors from step 152 (that serve as ground truth), and the predicted indices IP.

In some embodiments, the training process further comprises a fine-tuning phase 19.

In those embodiments, as shown on Figure 1, the module 11 is further configured to receive at least one machine learning(ML) model 29 and a specific training dataset 28 comprising labelled specific training EEG segments being specific to a downstream task. The fine-tuning phase 19 is based on the specific training dataset 28.

In some embodiments, when the representation 33 of the input EEG segment 32, obtained from the pretrained foundation model 31, is configured to be used as input of at least one machine learning model 29 related to a predefined task, the fine-tuning phase 19 comprises training the at least one machine learning model 29 upended to the pretrained foundation model 31, i.e. the encoder obtained from the second phase 15 based on self-supervised learning (or the second architecture 312 after the second phase 15). In other words, the at least one machine learning model 29 is a classification head. During the fine-tuning phase 19, the pretrained foundation model 31 and the at least one machine learning model 29 are trained as an ensemble. At the end of the fine-tuning phase 19, a fine-tuned machine learning model 35 is obtained. As represented on Figure 1, the fine-tuning phase 19 is implemented by the device 1. Alternatively, the fine-tuning phase 19 can be implemented by a device of similar internal components as the device 1, and having received and stored in memory the pretrained foundation model 31, but distinct from the device 1.

In some embodiments, during the fine-tuning phase 19, the weights of the pretrained foundation model 31 are frozen. The weights of the at least one machine learning model 29 may be first randomly initialized or being already pretrained on a generic task and then be trained on the corresponding specific training dataset 28. For example, early stopping, a regularization technique used to prevent overfitting during training, is used.

In other embodiments, during the fine-tuning phase 19, some of the weights of the pretrained foundation model 31 are left variable and trained concurrently to the weights of the at least one machine learning model 29. For example, early stopping, a regularization technique used to prevent overfitting during training, is used.

Once the training is completed, module 17 is configured to output the pretrained foundation model 31. If module 17 perform fine-tuning, it is further configured to output the fine-tuned at least one machine learning model 35. The pretrained foundation model 31 and at least one fine-tuned machine learning model 35may then be stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

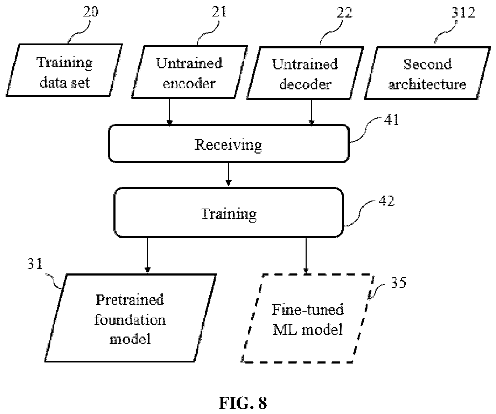

In its automatic actions, the device 1 may for example execute the following process (Figure 8):
- receiving the training dataset 20, the untrained encoder 21, the untrained decoder 22, and the second architecture 312 (step 41),
- implementing the first phase 14 and the second phase 15 using the training dataset 20 (step 42),
- optionally implementing the fine-tuning phase 19 using the specific training dataset 28 and the machine learning (ML) model 29.

The present invention also relates to a device 2 for obtaining a representation 33 of an input EEG segment 32 previously acquired, using the pretrained foundation model 31 obtained from the device 1, as described above. In other words, the device 2 is configured to perform inference using the previously trained, pretrained foundation model 31 obtained from the device 1, and optionally linked to a fine-tuned machine learning model 35 related to a specific downstream task. The device 2 will be described in reference to a particular function embodiment as illustrated in Figure 9.

The device 2 is adapted to receive as input the pretrained foundation model 31, already trained, and at least one input EEG segment 32.

The device 2 comprises a module 210 for receiving the pretrained foundation model 31 and the at least one input EEG segment 32, stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In advantageous embodiments, the pretrained foundation model 31 and all its parameters have been previously generated by a system including the device 2 for training. Alternatively, the trained artificial intelligence system of prediction 31 and its parameters are received from a communication network.

The at least one input EEG segment 32 comprises the EEG signals from multiple channels obtained by a subject.

For instance, the multiple channels can be a subset of channels from a 10-20 montage system. For instance, those multiple channels comprise the following channels: FP1, FP2, F7, F3, F4, F8, T7, C3, C4, T8, P7, P3, P4, P8, O1, and O2.

In some embodiments, the multiple channels comprise at least 16 channels with a minimal duration of 16 seconds.

In some embodiments, the minimal sampling frequency of the EEG signals from the multiple channels is 128 Hz.

The device 2 comprises at least one processor or a module 220 configured to feed the at least one input EEG segment 32 to the pretrained foundation model 31 so as to generate a representation 33 comprising a set of embedding vectors 34 of the at least one input EEG segment 32.

More in details, module 220 is configured to extract a set of input patches from said at least one input EEG segment 32, provide it as input to said pretrained foundation model 31 so as to obtain, as output, a set of indices associated to a set of codebook vectors corresponding to said set of input patches, and extract a set of embedding vectors using said set of indices and an updated codebook of the pretrained foundation model.

The device 2 may interact with a user interface, via which information can be entered and retrieved by a user. The user interface includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

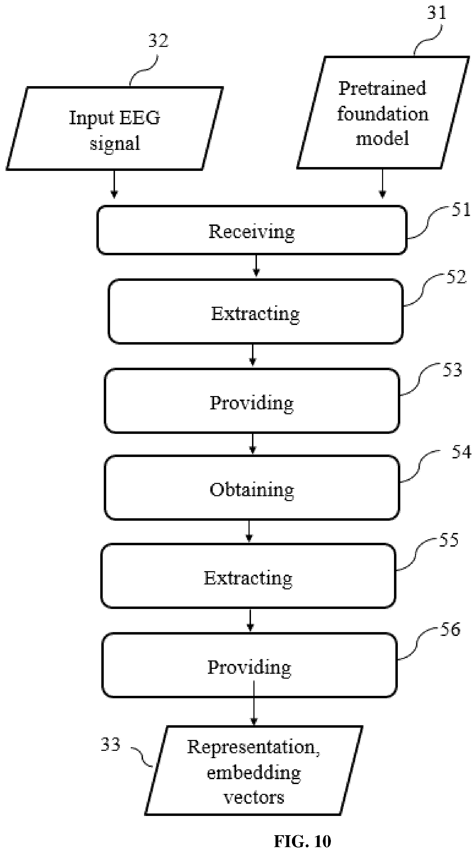

In its automatic actions, the device 2 may for example execute the following process (Figure 10):
- receiving the at least one input EEG segment 32 (step 51),
- extract a set of input patches from said at least one EEG segment (step 52);
- providing as input to said pretrained foundation model, said set of input patches, (step 53);
- obtain, as output, a set of indices associated to a set of codebook vectors corresponding to said set of input patches (step 54),
- extract a set of embedding vectors using said set of indices and an updated codebook of the pretrained foundation model 31 (step 55),
- provide the representation 33 of the input EEG segment 32, wherein the representation 33 of the at least one input EEG segment is defined as the set of embedding vectors 34 (step 56).

As previously explained, in some embodiments, the representation 33 and the set of embedding vectors 34 generated at step 53 are used as input of at least one machine learning model 29 fine-tuned on a predefined task.

Advantageously the fine-tuned machine learning model 35 obtained from the fine-tuning phase 19 and the machine learning model 29 is used.

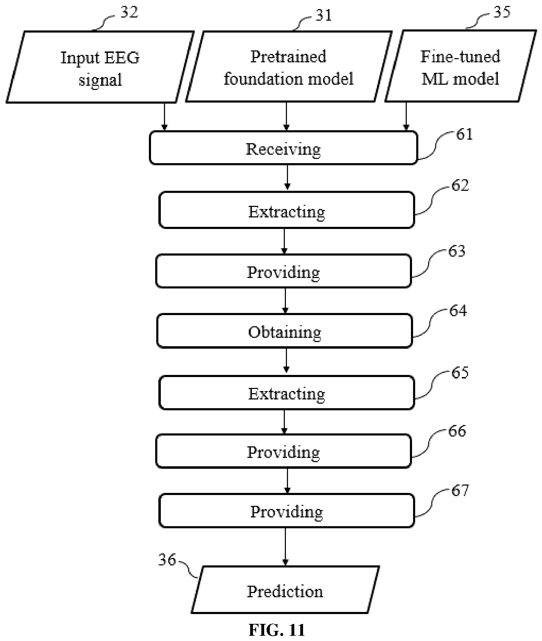

In those embodiments, in its automatic actions, the device 2, may further execute the following process (Figure 11):
- receiving at least one input EEG segment 32, the pretrained foundation model 31 and the fine-tuned machine learning model 35 (step 61),
- extracting a set of input patches 24 from the at least one input EEG segment 32 (step 62),
- providing, as input to the pretrained foundation model 31, the set of input patches 24 (step 63),
- obtaining, as output, a set of indices associated to a set of codebook vectors corresponding to the set of input patches 24 (step 64),
- extracting a set of embedding vectors using the set of indices and an updated codebook of the pretrained foundation model 31 (step 65),
- providing the set of embedding vectors to the fine-tuned machine learning model 35 so as to obtain a prediction 36 according to the predefined task (step 66),
- providing as output the prediction 36 of the predefined task (step 67).

A particular apparatus may embody the device 1 as well as the device 2 described above. It corresponds for example to a workstation, a laptop, a tablet, a smartphone, or a head-mounted display (HMD).

That apparatus is suited to obtention of a representation of an input EEG segment and to predictions according to predefined tasks, and to related ML training. It comprises the following elements, connected to each other by a bus of addresses and data that also transports a clock signal:
- a microprocessor (or CPU);
- a graphics card comprising several Graphical Processing Units (or GPUs) and a Graphical Random Access Memory (GRAM); the GPUs are quite suited to image processing and also to deep learning computations, due to their highly parallel structure;
- a non-volatile memory of ROM type;
- a RAM;
- one or several I/O (Input/Output) devices such as for example a keyboard, a mouse, a trackball, a webcam; other modes for introduction of commands such as for example vocal recognition are also possible;
- a power source; and
- a radiofrequency unit.

According to a variant, the power supply is external to the apparatus.

The apparatus also comprises a display device of display screen type directly connected to the graphics card to display synthesized images calculated and composed in the graphics card. According to a variant, a display device is external to the apparatus and is connected thereto by a cable or wirelessly for transmitting the display signals. The apparatus, for example through the graphics card, comprises an interface for transmission or connection adapted to transmit a display signal to an external display means such as for example an LCD or plasma screen or a video-projector. In this respect, the RF unit can be used for wireless transmissions.

When switched-on, the microprocessor loads and executes the instructions of the program contained in the RAM.

As will be understood by a skilled person, the presence of the graphics card is not mandatory, and can be replaced with entire CPU processing and/or simpler visualization implementations.

In variant modes, the apparatus may include only the functionalities of the device 1, and not the learning capacities of the device 2. In addition, the device 1 and/or the device 2 may be implemented differently than a standalone software, and an apparatus or set of apparatus comprising only parts of the apparatus may be exploited through an API call or via a cloud interface.

### MATERIALS AND METHODS

### Description of the pretrained foundation model 31:

The pretrained foundation model 31 is built in two main steps: (A) the continuous EEG signal is first encoded into a learnable compact representation through an EEG Tokenizer (first architecture 311) trained to reconstruct the power spectra of EEG patches using a Vector Quantized Variational Autoencoder (VQ-VAE), whose codebook is then (B) used by a second network to learn to predict the tokens associated to both masked and unmasked portions of the input EEG.

The goal of the EEG tokenizer (first architecture 311) is to learn a way to compress the input signal into a fixed number of meaningful features able to "summarize" its most relevant aspects in a way that they can generalize to unseen EEG data. These vectorized representations are called embeddings, because they aim to "embed" in themselves the minimal information needed to reconstruct some of the features characterizing the EEG (in this case, its power spectrum). As such, EEG tokenization is the process of projecting the continuous EEG input into a common lower-dimensional discrete subspace (the "codebook") that reduces the dimensionality of the input while preserving its more relevant inner relationships.

The embeddings used to encode the EEG are learned using a transformer-based VQ-VAE), a type of neural network that combines variational autoencoders with vector quantization to learn discrete latent representations of data (vqvae1, vqvae2). To do so, the VQ-VAE is trained to learn to encode the input data into a discrete codebook, i.e. a latent subspace of arbitrary shape that summarizes the most relevant information needed to reconstruct the power spectrum of the encoded EEG signal via a decoder block.

The learned codebook learned by the tokenizer is then used as ground-truth to train a Masked Token Predictor (MTP) deep transformer model (second architecture 312) to identify the codebook indices corresponding to the latent codebook vectors assigned to the masked EEG patches.

### Description of the first architecture 311:

The preprocessed EEG signals of each record is divided into non-overlapping, consecutive windows of x = {x_{i,j}∈ R^{T}x^{Nc} | i =1, 2, ..., T, j =1, 2, ..., Nc } , where T is the number of signal samples and NC the number of EEG channels in the window. The number of samples in the window is defined as T = TW * fs, being TW the length of the window (in seconds) and fs the sampling frequency of the signal. Each window thus stores a total of N_{P} = T_{W} * Nc patches, each patch composed by fs signal samples, p = {pⱼ,k ∈ R^{fs} | j =1, 2, ..., T_{W}, k =1, 2, ..., Nc } . This ensures input data could be fed into the same type of transformer architecture successfully used for image and signal processing (ViT, BIOT). Each patch is then passed to a temporal encoder block consisting of three consecutive convolutional layers with group normalization (groupNorm) and GELU activation functions (GELU) to extract temporal features with output embedding dimension D_{E} , resulting in e = {ej,k ∈ R^{DE} | j =1, 2, ..., T_{W}, k =1, 2, ..., Nc } vectors. Positional and channel information is then encoded into these feature tensors via an embedding layer implementing sinusoidal position encoding. Dropout (p=0.2) and normalization is then applied to the resulting embedding vectors, whose final shape is (N_{P}, D^{E}), **e** = {eₙ ∈ RDE | n =1, 2, ..., N_{P} }.

The embedding vectors are then fed to a sequence of 12 transformer blocks (each with 8 attention heads, hidden dimension of 1024 units and GELU activation function) which further enrich the encoding by using self-attention, a powerful mechanism that enables the model to learn both local and global relationships in the input data [transf]. The embedding vector eₙ corresponding to each patch p_{j,k} (originally representing 1 second of EEG signal for 1 channel) is then mapped to z = {zₙ∈ R^{DE} | n =1, 2, ..., N_{P} }. The shape of the whole encoded sequence being (N_{P}, D_{E}).

Then a codebook C = {vᵢ | i= 1, ..., C_{V} } ∈ R ^{Cv} x ^{CD}, a matrix of C_{V} discrete latent embedding vectors each defined by C_{D} elements, was initialized. These discrete vectors will be used to encode the latent representations characterizing the possible different power spectrum distributions of the EEG signal we want our model to be able to recognize and reconstruct. To do so, first, each encoded vector was projected in the sequence z from zₙ ∈ R^{DE} to zₙ ∈ R^{CD} (where n=1, ..., N_{P}) through two densely connected layers, then mapped each resulting zₙ to the nearest discrete latent embedding vector vi in the codebook based on cosine similarity, resulting in a quantized sequence with shape (N_{P}, C_{D}) q = {qₙ∈ R^{CD}| n =1, 2, ..., N_{P}}. Codebook updating was stabilized using the exponential moving average strategy and quantified by the commitment loss, a function that prevents the encoder's output from fluctuating too much between different codebook vectors during training (vqvae1).

After quantization, the set of discretized vector embeddings q corresponding to each patch in the input sequence are fed to the decoder, whose architecture mirrors the encoder but has reduced depth (3 transformer layers vs 12 in the encoder) and a final prediction head composed by 2 sequential fully connected layers that projects the output of the transformer block for the whole sequence to reconstruct the power spectra of all patches of the input sequence with shape (N_{P}, F), being F the number of spectral frequencies, **o** = {oₙ ∈ R^{F}| n =1, 2, ..., N_{P}}. The output o of the decoder will then be used to calculate the reconstruction loss against the target power spectrum of each patch, s = {sn ∈ RF| n=1, 2, ..., NP}, quantified using mean squared error (reconstruction loss = MSE(s, o)). The power spectrum distribution of each patch was estimated using Discrete Prolate Spheroidal Sequences (DPSS) multitaper windowing. The trained encoder block of the described VQ-VAE is what is referred to in the present disclosure to as "EEG tokenizer" (i.e. first architecture 311), as its output are the embedding vectors (the "tokens") encoding the EEG input patches.

### Description of the second architecture 312:

The pre-trained EEG Tokenizer (i.e. first architecture 311) described above is used to obtain the "ground-truth" that the masked token predictor model (i.e. second architecture 312) will be trained to predict using masked self-supervised learning. The preprocessed EEG signals are first segmented into patches and processed by the pre-trained tokenizer to obtain, for each input patch, the index corresponding to the associated latent vector in the codebook. The MTP is based upon the encoder architecture used in the VQ-VAE-based tokenizer described above. EEG signal is preprocessed and then segmented into a sequence of patches, which are later transformed via a temporal encoder layer to extract temporal feature tensors for each patch, e = {e_{j,k} | j =1, 2, ..., T_{W}, k =1, 2, ..., Nc }. A given portion r of these embeddings are randomly substituted via a learnable mask e_{M} ∈ R^{DE} token initialized with small random values drawn from a normal distribution, and the resulting tensor (storing both masked and non-masked patch embedding vectors) is then enriched via position and channel encoding using the same embedding layer architecture used by the tokenizer. The resulting embedding vectors are passed to a sequence of L transformer layers using GELU activation functions and varying number of attention heads and hidden units depending on the desired final model size, resulting in the output sequence z = {zₙ ∈ R^{DE} | n =1, 2, ..., N_{P} }. The output sequence is processed by a multi-layer perceptron head that first projects the transformer output for each patch into a Cv -dimensional vector of logits, h = {hₙ ∈ R^{Cv} | n =1, 2, ..., N_{P} }, which are then used to predict the index of the codebook vector associated to the largest logit for each patch. The whole MTP model (i.e. second architecture 312) is trained by minimizing the cross-entropy loss function between the true indices associated to each patch in the sequence (obtained from the codebook of the pre-trained tokenizer model) and the predicted ones for both the masked and the unmasked patches.

### Training dataset 20:

EEG records from two proprietary databases and four public datasets belonging to the TUH EEG Corpus, a well-known public database of clinical EEG records, were combined to build a pre-training dataset of 4000EEG hours, presented in Table 1. Bioserenity-Neurophy-FR1 is a database obtained in BioSerenity centers, ICUs, hospitals and private clinics in France from 2021 to 2024 (~45% female patients, mean age 63±22 years) that includes both clinically normal or altered EEG (sedation, epilepsy, encephalopathy, lesion, etc), recorded with either Micromed or BioSerenity's Neuronaute EEG system. BioSerenity-Neurophy-FR1 comprises fully anonymized long continuous EEG (24 to 75 hours) recorded using the Compumedics EEG system from US patients under epilepsy monitoring from 2021 to 2023. The four TUH datasets used to create the pretraining datasets were the "train" subset of TUH-Abnormal, TUH-Seizure, TUH-Events and TUH-Artifact. To avoid potential confounding effects, the relative percentage of files belonging to each database was kept constant across the pre-training datasets, as shown on Table 2. 6 years old was set as minimal age and record duration of at least 5 minutes was set as inclusion criteria. Records selected to build the pre-training datasets listed in Table 1 all belonged to the corresponding "train" subsets: no records from the "test" sets were included in pre-training BioSerenity-E1, i.e. the pretrained foundation model 31 according to the present disclosure. In Table 1, one token corresponds to one patch (1 second, 1 channel).

**Table 1: Pretraining Datasets composing the training dataset 20**

| Dataset | EEG hours | Windows | Tokens (i.e. codebook vectors) | Percentage |
|---|---|---|---|---|
| Bioserenity-Neurophy-FR1 | 2803 | 630,684 | 161455104 | 70% |
| Bioserenitv-US1 | 1201 | 270336 | 69206016 | 20% |
| TUH-Seizure | 200 | 45056 | 11534336 | 5% |
| TUH-Abnormal | 160 | 36044 | 9227264 | 4% |
| TUH-Events | 28 | 6308 | 1614848 | 0.7% |
| TUH-Artifacts | 12 | 2,703 | 691968 | 0.3% |
| Complete Dataset | 4005 | 901120 | 230686720 | 100% |

### Specific training dataset 28:

The pretrained foundation models were tested on different downstream tasks from 4 finetuning datasets presented in Table 2. The continuous EEG signals of all records in each of these datasets were first divided into non-overlapping windows of 16 seconds (each storing the signal of 16 channels), and each window was then assigned with a unique class label to predict. Neurophy-Abnormal and Neurophy-Multiclass are a subset of the larger BioserenityNeurophy-FR1 clinical database composed by 338 hours of EEG from 7536 records reviewed by an expert neurologist and labelled as either normal or representative of three broad types of abnormality (lesion, status epilepticus, encephalopathy). TUH-Abnormal is a balanced dataset containing 1004 hours of EEG from records classified as clinically normal or abnormal that was drawn from [tuab]. TUH-Seizure contains 138 hours of EEG from records with annotated seizure events and normal background activity. In Table 2, one token corresponds to one patch (16 channels, 16 seconds). Every window including a seizure event of at least 3 seconds was assigned the label "Seizure".

**Table 2: Fine-tuning datasets**

| **Dataset** | **Subset** | **EEG hours (h)** | **Windows** | **Tokens** | **Size (GB)** | **Labels (windows, %)** |
|---|---|---|---|---|---|---|
| Neurophy-Abnormal | Train | 270 | 60,850 | 15,5M | 20 | Normal (31050,51) |
| | | | | | | Abormal (29800,49) |
| | Test | 68 | 15,285 | 3,9M | 5 | Normal (7800,51) |
| | | | | | | Abormal (7485,49) |
| Neurophy-Multiclass | Train | 270 | 60,850 | 15,5M | 20 | Normal (31050,51) |
| | | | | | | Lesion (11100,18) |
| | | | | | | Status (9670,16) |
| | | | | | | Encephalopathy (9030,14) |
| | test | 68 | 15,282 | 3,9M | 5 | Normal (7800,51) |
| | | | | | | Lesion (2730,18) |
| | | | | | | Status (2415,16) |
| | | | | | | Encephalopathy (2340,14) |
| TUH-Abnormal | Train | 904 | 203,388 | 52M | 65 | Normal (101257,49) |
| | | | | | | Abormal (102131,51) |
| | test | 102 | 23,040 | 5,8M | 7,4 | Normal (12412,54) |
| | | | | | | Abormal (10628,46) |
| TUH-Seizure | Train | 106 | 23,840 | 6,1M | 7,7 | Background (12000,51) |
| | | | | | | Seizure (11840,49) |
| | test | 32 | 7,152 | 1,8M | 2,3 | Background (5121,71) |
| | | | | | | Seizure (2031,29) |

### Training:

As previously described, the pretrained foundation model 31 is obtained via a training process 42 comprises a combination of a first phase 14 and a second phase 15, using self-supervised representation learning via EEG quantization and masking part of the input EEG signal. Then, when a machine learning model 29, corresponding to a downstream specific task such as normal vs. abnormal EEG classification, disease prediction and seizure detection, is upended to the pretrained foundation model 31, a fine-tuning phase 19 is implemented based on specific supervised learning objectives associated to this specific task.

### Detailed training strategy of the first phase 14:

The first architecture 311 **(tokenizer)** was trained for 100 epochs using AdamW optimizer with a cosine learning rate scheduler using batch size of 64. The tokenizer optimizes a loss function defined as the sum of both the spectrum reconstruction loss and the commitment loss from vector quantization. To assess how much the tokenizer exploited its representation capacity during pre-training, codebook usage percentage, a measure of the proportion of codebook vectors that are actually used, on one hand, and normalized codebook entropy, which measures how well-distributed those used vectors are distributed, on another hand, were quantified. The transformer-based VQ-VAE first architecture 311 has 12.6M learnable parameters and was pre-trained on 4000 EEG hours (see Table 1).

Table 3 summarizes the hyperparameters used during the first phase 14 to train the first architecture 311 **(tokenizer).**

**Table 3: First architecture hyperparameters**

| | Hyperparameters | Values |
|---|---|---|
| Temporal encoder | 2D-CNN Depth | 3 |
| | Input channels | {1,16,16} |
| | Output channels | {16,16,16} |
| | Kernel size | {11,3,3} |
| | Input stride | {8,1,1} |
| | Input padding | {5,1,1} |
| | Activation function | GELU |
| Position & Channel Embedding | Num. time patches (T_{W}) | 16 |
| | Num. channel patches (N_{C}) | 16 |
| | Embedding dimension (D_{E}) | 256 |
| Transformers | Encoder depth | 12 |
| | Decoder depth | 3 |
| | Attention heads | 8 |
| | MLP size | 1024 |
| | Activation Function | GELU |
| Quantizer | Codebook vectors (C_{V}) | 8192 |
| | Codebook dimension (C_{D}) | 64 |
| | Beta | 0.3 |
| | Decay | 0.98 |
| Training | Training size | 4000 EEG hours |
| | Batch size | 128 |
| | Peak learning rate | 0.00032 |
| | Initial learning rate | 0.000075 |
| | Minimal learning rate | 1e-5 |
| | Learning Rate Scheduler | Cosine |
| | Optimizer | AdamW |
| | AdamW Betas | (0.9,0.95) |
| | Weight decay | 0.01 |
| | Total epochs | 100 |
| | Warmup epochs | 10 |
| | Loss Function | Commit Loss + Reconstruction Loss |

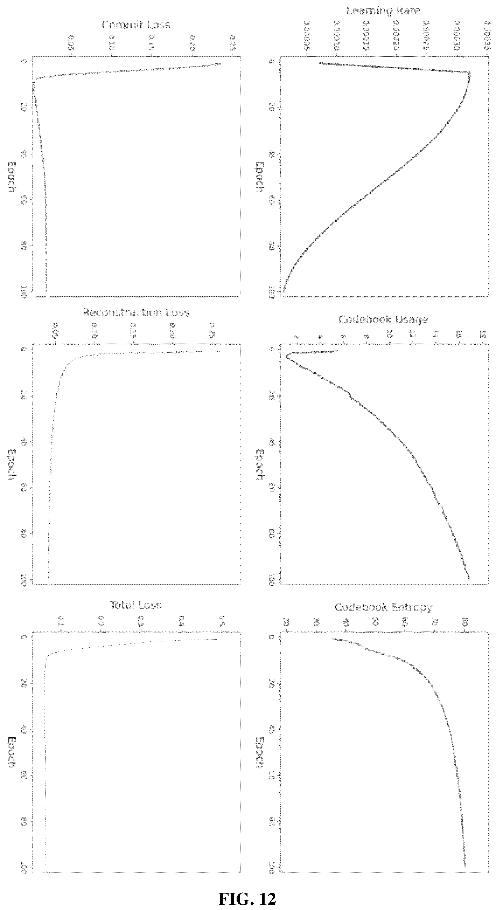

**Figure 12** shows training metrics related to the first phase 14. From top-left to bottom-right: learning rate, codebook usage percentage, codebook, normalized entropy, commit loss, reconstruction loss, total loss. Tokenizer backpropagation is based on total loss.

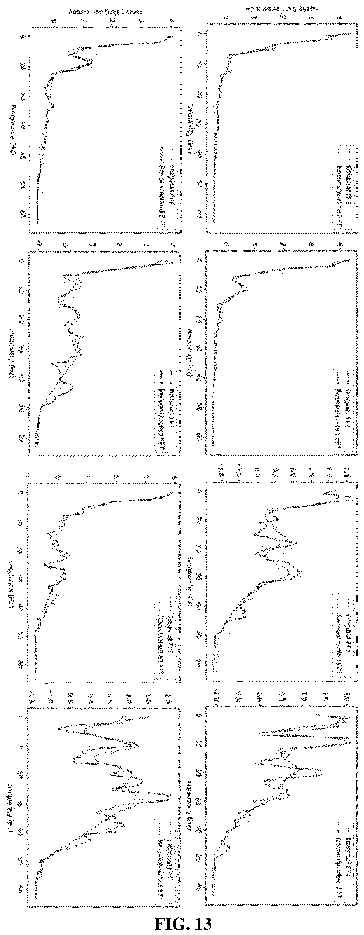

**Figure 13** shows a comparison between original spectra of patches and spectra reconstructed by the first architecture 311 trained after the first phase 14 (overlayed). Each subplot corresponds to the power spectrum distribution obtained from 1-second EEG window of one channel (i.e. one patch).

### Detailed training strategy of the second phase 15:

The second architecture 312 **(MTP)** was also trained for 30 epochs using AdamW optimizer with a cosine learning rate scheduler using batch size of 128. The second architecture 312 is trained to minimize the cross-entropy loss between the predicted codebook indices assigned to masked and unmasked input patches and the true indices assigned to the unmasked input patches by the pretrained tokenizer's encoder. As additional performance metric of the second phase 15, the accuracy over epochs between the predicted and the true codebook indices was also quantified. The second architecture 312 has 11.7 M learnable parameters and was pretrained on 4000 EEG hours (see Table 1).

Table 4 summarizes the hyperparameters used during the second phase 15 to train the second architecture 312 **(masked token predictor).**

**Table 4: Second architecture hyperparameters**

| | Hyperparameters | Values |
|---|---|---|
| Temporal Encoder | CNN Depth | 3 |
| | Input channels | {1,16,16} |
| | Output channels | {16,16,16} |
| | Kernel size | {11,3,3} |
| | Input stride | {8,1,1} |
| | Input padding | {5,1,1} |
| | Activation function | GELU |
| Position & Channel Embedding | Num. time patches (T_{W}) | 16 |
| | Num. channels patches (N_{C}) | 16 |
| | Embedding dimension (D_{E}) | 256 |
| Transformers | Encoder depth (L) | 12 |
| | Attention heads | 16 |
| | MLP size | 1024 |
| | Activation Function | GELU |
| Training | Training size | 4000 EEG hours |
| | Batch size | 128 |
| | Peak learning rate | 0.0032 |
| | Initial learning rate | 8e-05 |
| | Minimal learning rate | 8e-05 |
| | Learning Rate Scheduler | Cosine |
| | Optimizer | AdamW |
| | AdamW Betas | (0.9,0.95) |
| | Weight decay | 0.01 |
| | Total epochs | 30 |
| | Warmup epochs | 5 |
| | Gradients clipping | 1.0 |
| | Mask ratio | 0.7 |
| | Loss Function | Cross-Entropy |

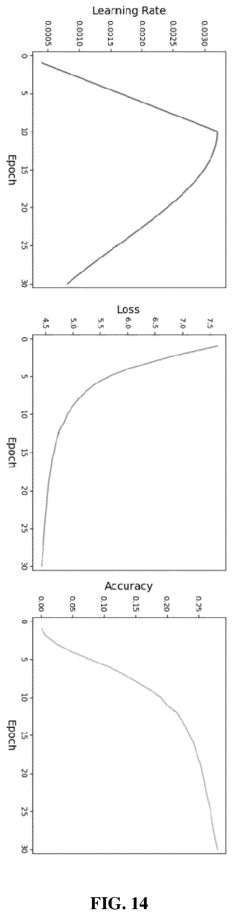

Figure 14 shows training metrics related to the second phase 15. From top-left to bottom-right: learning rate, cross-entropy loss, accuracy in predicting the correct codebook indices.

### Fine-tuning phase 19 details:

The pretrained foundation model 31 was tested on multiple downstream tasks to evaluate generalizability of the learned representations. To do so, a pretrained foundation model 31 with frozen weights was used as base model, to which a classifier head with randomly initialized weights, that was trained on the corresponding downstream training dataset using early stopping, was added. The classifier head was composed of three convolutional layers and an average pooling layer. The ensemble formed by the pretrained foundation model 31 and the classifier head was trained via supervised learning to predict the unique label associated to each input window of the corresponding downstream training dataset. The finetuning phase 19 was performed using early stopping (patience = 5 epochs), batch size of 32, Adam optimizer and constant learning rate (LR_{head}). Binary cross-entropy was used as loss function for Neurophy-Abnormal, TUH-Abnormal and TUH-Seizure and cross-entropy was used as loss function for Neurophy-Multiclass. To assess the variability of results due to randomness in fine-tuning, three finetuning jobs were run for each model, each downstream task.

Table 5 presents the detailed parameters of the implementation of the fine-tuning phase 19.

**Table 5: Fine-tuning phase parameters**

| | Neurophy-Abnormal | TUH-Abnormal | TUH-Seizure | Neurophy-Multiclass |
|---|---|---|---|---|
| Classes | 2 | 2 | 2 | 4 |
| Batch size | 32 | 32 | 32 | 32 |
| Learning rate | 1e-04 | 1e-04 | 1e-04 | 1e-04 |
| Patience | 5 | 5 | 5 | 5 |
| Optimizer | Adam | Adam | Adam | Adam |
| Loss function | Binary Cross-Entropy | Binary Cross-Entropy | Binary Cross-Entropy | Cross-Entropy |

### RESULTS

Model building and training were implemented using PyTorch with BF16 floating-point precision. The first architecture 311 **(tokenizer)** is characterized by 12.6M learnable parameters and was trained on 4000 hours of EEG data using distributed data parallel on 4 NVIDIA A10G GPUs for 100 epochs with a batch size of 128. Tokenization training took 6.7 hours.

The second architectures 312 (masked-token predictor model) has 11.7 M learnable parameters and was trained on 4000 hours of EEG data and using distributed data parallel on 16 NVIDIA A10G GPUs for 30 epochs with varying batch size of 128. The entire masked-token prediction training processes took from 3.5 hours.

Fine-tuning was performed on a single NVIDIA A10G GPU; the batch size was set to 32, with the total training time per epoch ranging from 2 to 5 minutes, depending on the task.

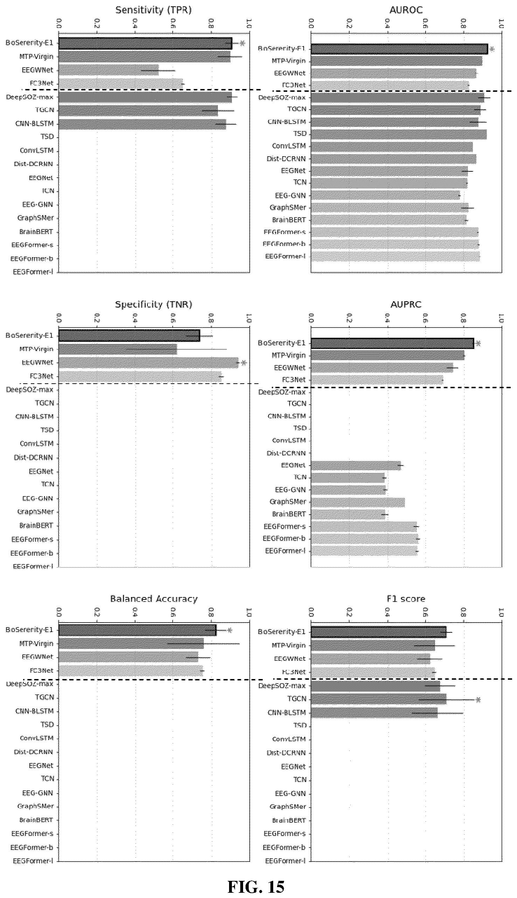

Figure 15 to 18 show comparison of results of downstream tasks with and without the use of the representation 33 obtained with the pretrained foundation model 31.

More in details, Figure 15 shows the corresponding comparison for a downstream task consisting of seizure detection. The performance of the pretrained foundation mode 31 was evaluated against several baseline models on the seizure detection task using the TUH-Seizure dataset. Results indicate that the pretrained foundation model 31 consistently outperformed other models across key metrics. Specifically, it achieved the highest AUROC, AUPRC, Sensitivity (TPR), and Balanced Accuracy, as highlighted in the plots (stars indicate top performance). The model also demonstrated competitive F1-scores and Specificity (TNR), maintaining a robust balance between true positive and true negative rates. These results underscore the effectiveness of the pretrained foundation model 31 according to the present disclosure as a foundation model for EEG-based seizure detection tasks. Error bars in the plots reflect variability (standard deviations) across multiple runs, further validating the model's reliability. Models represented on the left-hand side of the dashed black lines were run as baseline models to evaluate the pretrained foundation model 31 according to the present disclosure, whereas results of the models on the right-hand side of the dashed black lines represent the state-of-the-art obtained from the literature for binary seizure detection.

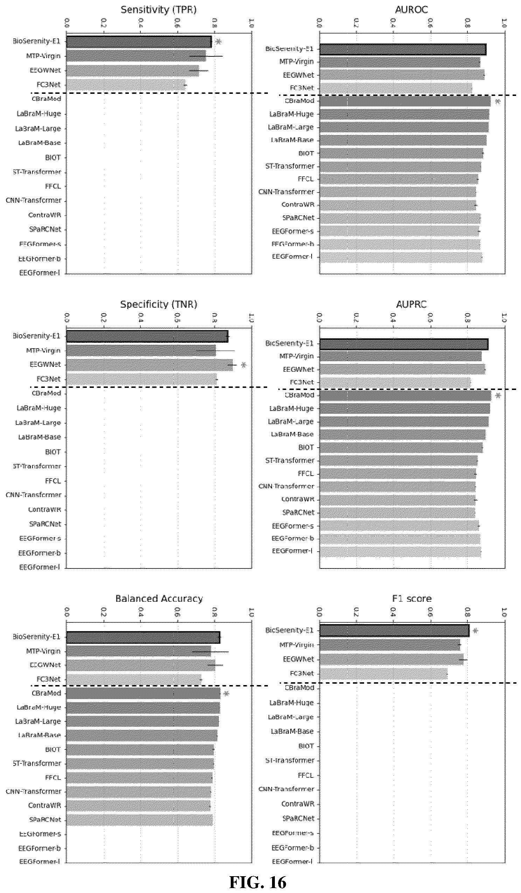

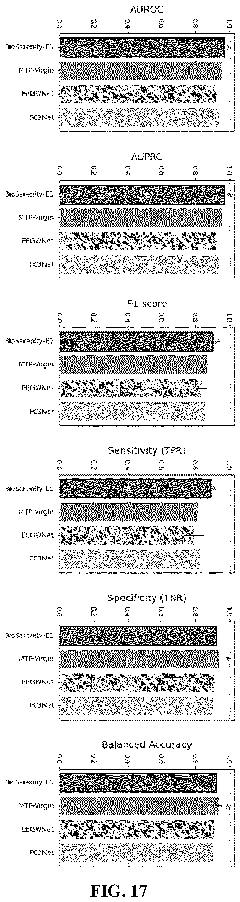

Figures 16 and 17 show the corresponding comparisons for a downstream task consisting of normal vs. abnormal EEG classification. The performance of the pretrained foundation model 31 according to the present disclosure in correctly distinguishing normal against clinically abnormal EEG was evaluated using two datasets: TUH-Abnormal and Neurophy-Abnormal. On TUH-Abnormal (Figure 16), the pretrained foundation model 31 according to the present disclosure consistently ranks in top-tier across all metrics, demonstrating competitive performance. The pretrained foundation model 31 according to the present disclosure excels in sensitivity (TPR) and weighted F1 score, highlighting its ability to correctly identify abnormal EEGs and maintain a strong balance between precision and recall. Models represented on the left-hand side of the dashed black lines were run as baseline models to evaluate our model, whereas results of the models represented on the right-hand side of the dashed black lines represent the state-of-the-art obtained from the literature for binary seizure detection.

The pretrained foundation model 31 according to the present disclosure exhibited also excellent classification performances on the proprietary Neurophy-Abnormal dataset (Figure 17), scoring second to MTP-Virgin only on Specificity (TNR) and Balanced Accuracy, showing however a small percentage difference and displaying higher stability, as showed by decreased inter-trial variability compared to MTP-Virgin.

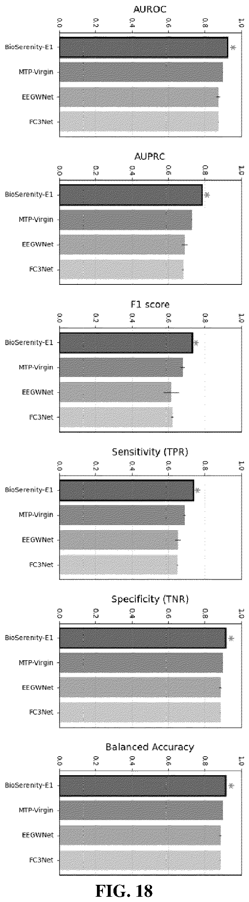

Figure 18 shows the corresponding comparisons for a downstream task consisting of multi-class EEG classification using the proprietary Neurophy-Multiclass dataset and compared against three internal baseline models: MTP-Virgin, EEGWNet, and FC3Net. Each window in this dataset is assigned to a unique label, that the models are trained to predict. The target classes are: "normal", "status epilepticus", "lesion" and "encephalopathy". As shown in Figure 18, the pretrained foundation model 31according to the present disclosure demonstrated superior performance across all evaluated metrics (asterisks mark best result): it achieved the highest AUROC, indicating its robust ability to distinguish between classes. The largest improvements compared to the other baseline models are seen in AUPRC and F1 score, highlighting the effectiveness of the pretrained foundation model 31 according to the present disclosure in handling imbalance in the training data and optimizing precision-recall trade-offs, two important aspects to control when designing algorithms to support clinical applications. State-of-the-art performances from the literature are not available as "Neurophy-Multiclass" is a proprietary dataset.

State-of-the-art values are not present for downstream tasks on the "Neurophy" dataset as it is a proprietary dataset.

### Performances in low-data regimes

One of the most limiting factors in the development of performant clinical AI algorithms is data scarcity, either because obtaining enough data of good quality and annotations can be expensive and time-consuming, or because the prevalence of the condition under study is low (e.g. in the case of rare diseases) and therefore the available data volumetry on which to train is intrinsically small. To assess the utility the pretrained foundation model 31 according to the present disclosure in low-data regimes, the fine-tuning phase 19 was further implemented only on increasing fractions of the available training data. The obtained performance against the full test set on the downstream tasks was quantified. The results are shown on Figure 16). On Figure 16: mtp corresponds to the pretrained 12M masked token predictor model (i.e. the pretrained foundation model 31 according to the present disclosure); mtp-virgin is the same architecture of the pretrained foundation model 31 according to the present disclosure but trained from scratch using the available training data, as is eeg-net. A positive impact of using the pretrained foundation model 31 can be observed in low-data regimes.

### Evaluation metrics

In the present disclosure, a number of metrics was adopted to assess performance on both binary and multi-class classification downstream tasks. "Accuracy" is the ratio of correctly predicted instances to the total number of instances. "Sensitivity", also known as the True Positive Rate, evaluates a model's ability to correctly identify positive cases. It measures the proportion of actual positive cases that were correctly identified. "Specificity", also referred to as True Negative Rate, measures the model's ability to correctly identify negative cases, calculated as the ratio of true negatives to all negative outcomes. This metric is particularly important when there's a high cost associated with false positives. The Area Under the Receiver Operating Characteristic curve ("AUROC") measures the overall model's discriminatory ability in terms of True Positive Rate and False Positive Rate assessed on a range of classification thresholds. Values range from 0.5 (random guess) to 1.0 (perfect classification). "AUPRC" is a performance metric calculating the area under the Precision-Recall (PR) curve obtained over a range of classification thresholds. It summarizes the trade-off between Precision (accuracy of positive predictions) and Recall (ability to find all positive cases). "Weighted F1" is the harmonic mean of Precision and Recall that accounts for class imbalance by weighting each class's F1 score based on its frequency in the dataset. Together with AUPRC, the weighted F1 score provides a robust measure in multi-class classification settings, where different classes may have different prevalence.

### Conclusions

The pretrained foundation model 31 presented in the present disclosure has been originally developed to be used on clinical use-cases utilizing the standard 10-20 EEG system; with this in mind, the largest list of EEG channels was selected from the available databases, to maximize the number of total EEG hours that we could use for a training process. The presented results are obtained using models trained on that list of 16 channels.

## Claims

1. A device (1) for generating a pretrained foundation model (31) for obtaining a representation (33) of an input EEG segment (32) previously acquired, said device comprising:
- at least one input configured to receive:
- a training dataset (20) comprising a plurality of EEG segments (32) previously acquired, each EEG segment (32) comprising the EEG signals from multiple channels obtained by a subject from plurality of subjects,
- an untrained encoder (21), being associated to a codebook (23), configured to receive as input a set of input patches associated to an input EEG segment (32) and to provide as output a set of embedding vectors each representative of one input patch in a latent representation space, said set of input patches being extracted from said input EEG segment (32),
- a untrained decoder (22) configured to receive as input a set of codebook vectors (260) assigned to said set of embedding vectors associated to said input EEG segment (32) and to output a set of power spectra corresponding to said set of input patches,
- at least one processor configured to generate said pretrained foundation model (31) via a training process based on said training dataset,
- at least one output configured to provide as output said pretrained foundation model (31),
wherein said training process comprises:
- obtaining a pretrained encoder (PE) and an updated codebook (UC) by training the untrained encoder (21) and untrained decoder (22) and updating the codebook using said training dataset (20),
- obtaining said pretrained foundation model (31) by performing self-supervised learning using said training dataset (20), said untrained encoder (312), said pretrained encoder (PE), said updated codebook (UC) and a predefined loss function;
wherein the pretrained foundation model (31) comprises the untrained encoder (312) with updated weights obtained at the end of the self-supervised learning and wherein said representation (33) of the at least one input EEG segment (32) is defined as the set of embedding vectors.

2. The device (1) according to claim **1,** wherein obtaining a pretrained encoder (PE) and an updated codebook (UC) by training the untrained encoder (21) and untrained decoder (22) and updating the codebook (23) using said training dataset (20), comprises performing iteratively until convergence, for each EEG segment of said plurality of EEG segments:
- obtaining a set of patches (240), each patch being extracted from the EEG segment;
- providing said set of patches (240) as input to said untrained encoder (21) and obtaining as output a set of embedding vectors (250);
- mapping said set of embedding vectors (250) to the codebook (23) so to obtain the associated set of codebook vectors (260) using quantization;
- decoding with the untrained decoder (22) said set of codebook vectors (260), and
- updating the codebook (23) and updating weights of the untrained encoder (21) and the untrained decoder (22).

3. The device (1) according to either one of claim **1** or **2,** wherein obtaining said pretrained foundation model (31) by performing self-supervised learning comprises performing iteratively until convergence, for each EEG segment of said plurality of EEG segments:
- obtaining an unmasked set of patches, each patch of the unmasked set of patches being extracted from the EEG segment;
- providing said unmasked set of patches as input to said pretrained encoder (PE) and obtaining as output a set of target encoded patches and mapping them to the updated codebook (UC) obtaining a set of target indices (IT) associated to a set of target codebook vectors associated to the EEG segment;
- obtaining a masked set of patches by masking at least one patch of said unmasked set of patches;
- providing said masked set of patches as input to said untrained encoder and obtaining as output a set of predicted encoded patches and mapping them to the updated codebook obtaining a set of predicted indices (IP) associated to a set of predicted codebook vectors associated to the EEG segment;
- updating weights of the untrained encoder (312);
wherein said predefined loss function is configured to compare the set of target indices (IT) with the set of predicted indices (IP),
wherein the pretrained foundation model (31) comprises the untrained encoder (312) with said updated weights obtained at the end of the self-supervised learning.

4. The device (1) according to any one of claims **1** to **3,** wherein said representation (33) of said input EEG segment is configured to be used as input of at least one machine learning model (29) related to a predefined task, said at least one predefined task being one among:
- a discrimination of said input EEG segment between a normal EEG segment and an abnormal EEG segment,
- an anesthesia depth determination,
- a pathology classification task,
- a sleep staging task,
- seizure detection task,
- EEG biomarker identification.

5. The device (1) according to claim **4,** wherein:
- the at least one input is further configured to receive a specific training dataset (28) comprising labelled specific training EEG segments being specific to said downstream task,
- the training process further comprises a fine-tuning phase (19) based on said specific training dataset (28).

6. The device (1) according to claim **5**,wherein:
- said untrained encoder (21) comprises an architecture comprising convolutional layers (2110), time and channel encoding (2120, 2130) and several transformer blocks (2140), each transformer block comprising attention heads and,
- the fine-tuning phase (19) comprises training said at least one machine learning model (29) upended to said pretrained foundation model (31), which is used as base model, so as to obtain a fine-tuned machine learning model (35).

7. The device (1) according to any one of the preceding claims, wherein said untrained encoder (21) and untrained decoder (22) are comprised in a vector-quantization variational autoencoder architecture.

8. A device (2) for obtaining a representation (33) of an input EEG segment (32), by using a pretrained foundation model (31) obtained with a device (1) according to any one of claims **1** to **7,** said device (2) comprising:
- at least one input configured to receive:
• at least one input EEG segment (32);
- at least one processor configured to:
• extract a set of input patches from said at least one EEG segment (32);
• provide, as input to said pretrained foundation model (31), said set of input patches,
• obtain, as output, a set of indices associated to a set of codebook vectors corresponding to said set of input patches,
• extract a set of embedding vectors using said set of indices and an updated codebook of the pretrained foundation model,
- at least one output configured to provide said representation (33) of said input EEG segment (32), wherein said representation (33) of the at least one input EEG segment (32) is defined as said set of embedding vectors (34).

9. A computer-implemented method for generating a pretrained foundation model (31) for obtaining a representation (33) of an input EEG segment (32) previously acquired, said method comprising:
- receiving:
- a training dataset (20) comprising a plurality of EEG segments previously acquired, each EEG segment comprising the EEG signals from multiple channels obtained by a subject from plurality of subjects,
- an untrained encoder (21), being associated to a codebook (23), configured to receive as input a set of input patches (240) associated to an input EEG segment and to provide as output a set of embedding vectors (250) each representative of one input patch in a latent representation space, said set of input patches (240) being extracted from said input EEG segment,
- a untrained decoder (22) configured to receive as input a set of codebook vectors (260) assigned to said set of embedding vectors (250) associated to said input EEG segment and to output a set of power spectra (270) corresponding to said set of input patches (240),
- at least one processor configured to generate said pretrained foundation model (31) via a training process based on said training dataset (20),
- providing as output said pretrained foundation model (31),
wherein said training process comprises:
- obtaining a pretrained encoder (PE) and an updated codebook (UC) by training the untrained encoder and untrained decoder and updating the codebook using said training dataset, by performing iteratively until convergence, for each EEG segment of said plurality of EEG segments:
- obtaining a set of patches (240), each patch being extracted from the EEG segment;
- providing said set of patches (240) as input to said untrained encoder (21) and obtaining as output a set of embedding vectors (250);
- mapping said set of embedding vectors (250) to the codebook (23) so to obtain the associated set of codebook vectors (260) using quantization;
- decoding with the untrained decoder (22) said set of codebook vectors (260), and
- updating the codebook (23) and updating weights of the untrained encoder (21) and the untrained decoder (22);
- obtaining said pretrained foundation model (31) by performing self-supervised learning using said training dataset (20), said untrained encoder (312), said pretrained encoder (PE), said updated codebook (UC) and a predefined loss function; wherein said self-supervised learning comprises performing iteratively until convergence, for each EEG segment of said plurality of EEG segments:
- obtaining an unmasked set of patches, each patch of the unmasked set of patches being extracted from the EEG segment;
- providing said unmasked set of patches as input to said pretrained encoder (PE) and obtaining as output a set of target encoded patches and mapping them to the updated codebook obtaining a set of target indices (IT) associated to a set of target codebook vectors associated to the EEG segment;
- obtaining a masked set of patches by masking at least one patch of said unmasked set of patches;
- providing said masked set of patches as input to said untrained encoder (312) and obtaining as output a set of predicted encoded patches and mapping them to the updated codebook obtaining a set of predicted indices (IP) associated to a set of predicted codebook vectors associated to the EEG segment;
- updating weights of the untrained encoder (312);
wherein said predefined loss function is configured to compare the set of target indices (IT) with the set of predicted indices (IP);
wherein the pretrained foundation model (31) comprises the untrained encoder (312) with the updated weights obtained at the end of the self-supervised learning.

10. A computer-implemented method for obtaining a representation (33) of an input EEG segment (32), by using the pretrained foundation model (31) obtained with the method according to the preceding claim, said method comprising:
- receiving (51):
- at least one EEG segment,
- extracting (52) a set of input patches from said at least one EEG segment;
- providing (53), as input of said pretrained foundation model (31), said set of input patches;
- obtaining (54) as output, a set of indices associated to a set of codebook vectors corresponding to said set of input patches;
- extracting (55) a set of embedding vectors (34) using said set of indices and an updated codebook of the pretrained foundation model (31);
- providing (56) said representation (33) of said input EEG segment (32), wherein said representation (33) of the at least one input EEG segment (32) is defined as said set of embedding vectors (34).

11. A computer-implemented method for predicting according to a predefined task using a pretrained foundation model (31) obtained from a method according to claim 8, and a fine-tuned machine learning model (35), obtained from the device (1) according to either one of claim 5 or 6, wherein the method comprises:
- receiving (61) at least one EEG segment;
- extracting (62) a set of input patches from said at least one EEG segment;
- providing (63), as input to said pretrained foundation model (31), said set of input patches,
- obtaining (64), as output, a set of indices associated to a set of codebook vectors corresponding to said set of input patches,
- extracting (65) a set of embedding vectors using said set of indices and an updated codebook of the pretrained foundation model (31),
- providing (66) said set of embedding vectors to said fine-tuned machine learning model so to obtain a prediction (36) according to said predefined task, providing (67) as output said prediction (36) of the predefined task

12. The method according to claim **11,** wherein said at least one predefined task is one among:
- a discrimination of said input EEG segment between a normal EEG segment and an abnormal EEG segment,
- an anesthesia depth determination,
- a pathology classification task,
- a sleep staging task,
- seizure detection task,
- EEG biomarker identification.

13. Use of a representation (33) of an input EEG segment (32) obtained with the method according to claim **10,** as input of a downstream task being a discrimination of said input EEG segment between a normal segment and an abnormal segment.

14. Use of a representation (33) of an input EEG segment (32) obtained with the method according to claim **10,** as input of a downstream task being a pathology classification task.

15. A computer program product comprising instructions which, when the program is executed by a computer, cause the computer to carry out the method according to any one of claims **7** to **10.**
